Europäisches Patentamt

European Patent Office

Office européen des brevets

(19)

(11) Veröffentlichungsnummer: **0 256 285 B1**

(12) # EUROPÄISCHE PATENTSCHRIFT

(45) Veröffentlichungstag der Patentschrift: **20.03.91**

(51) Int. Cl.⁵: **A61K 9/10**

(21) Anmeldenummer: **87109765.5**

(22) Anmeldetag: **07.07.87**

(54) Pharmazeutische Formulierung und Verfahren zu deren Herstellung.

(30) Priorität: **11.07.86 DE 3623376**

(43) Veröffentlichungstag der Anmeldung:
**24.02.88 Patentblatt 88/08**

(45) Bekanntmachung des Hinweises auf die
Patenterteilung:
**20.03.91 Patentblatt 91/12**

(84) Benannte Vertragsstaaten:
**AT BE CH DE ES FR GB GR IT LI LU NL SE**

(56) Entgegenhaltungen:
**EP-A- 0 212 875**
**DE-A- 3 225 706**
**GB-A- 1 155 036**

(73) Patentinhaber: **BEHRINGWERKE Aktiengesellschaft
Postfach 1140
W-3550 Marburg 1(DE)**

(72) Erfinder: **List, Paul Heinz, Prof. Dr. Dr.
Auf'm Gebrande 23
W-3550 Marburg(DE)**
Erfinder: **Schmidt, Peter Christian, Prof. Dr.
Am Hasenküppel 31
W-3550 Marburg(DE)**
Erfinder: **Steffens, Klaus-Jürgen, Dr.
Schwanallee 1
W-3550 Marburg(DE)**
Erfinder: **Perschbacher, Harald
Lorscher Strasse 9
W-6380 Bad Homburg(DE)**
Erfinder: **Kraemer, Hans Peter, Dr.
Birkenweg 16
W-3550 Marburg(DE)**
Erfinder: **Sedlacek, Hans Harald, Dr.
Sonnenhang 3
W-3550 Marburg(DE)**

(74) Vertreter: **Klein, Otto, Dr. et al
Hoechst AG Zentrale Patentabteilung Postfach 80 03 20
W-6230 Frankfurt am Main 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine pharmazeutische Formulierung zur parenteralen Verabreichung von in physiologisch verträglichen, hydrophilen und lipophilen Medien sehr schwer löslichen Substanzen, welche durch Dispergierung der feinstvermahlenen Substanz in der Ölphase, anschliessende Emulgierung und Homogenisierung hergestellt wird.

Die Herstellung parenteral applizierbarer Emulsionen wird beispielweise von Hansrani (P.K. Hansrani, S.S. Davis, M.J. Groves, J. Parenter, Sci. Technol., 37 , 145 (1983)) beschrieben. Dabei wird ein vegetabilisches Öl mittels eines Emulgators, meistens Lecithin, in Wasser emulgiert und durch Hochdruckhomogenisierung eine Partikelgröße von weniger als einem Mikrometer erreicht. Bei diesem Verfahren liegt als Endprodukt ein System flüssig/flüssig vor.

Es sind zahlreiche Rezepturen zur intravenösen Verabreichung schwer löslicher Substanzen beschrieben. Man kann die Substanzen beispielweise mittels Kosolventien in Lösung bringen oder in Mizellen solubilisieren (Techniques of Solubilizing Drugs, S.H. Yalkowsky ed., 1981, Marcel Dekker, New York, S. 15-134). Diese beiden Möglichkeiten bieten hinsichtlich der Verträglichkeit deutliche Nachteile gegenüber den Emulsionsformulierungen, die den Wirkstoff, beispielsweise Diazepam (Handelspräparat Diazemuls®, Kabivitrum, BRD seit 1.1.1983 im Handel) in der Ölphase gelöst enthalten (A.S. Olesen, M.S. Hüttel, Br. J. Aneasth. 52 , 609, (1980)).

Andere Verfahren nutzen die parenteralen Nähremulsionen als Vehikel und fügen die Substanz in einem Kosolvens gelöst zu, was bei intravenöser Verabreichung zu Komplikationen führen kann (A.J. Repta, Topics in Pharmaceutical Sciences, D.D. Breimer und P. Speiser ed., 1981, S. 131, Elsevier/North-Holland Biomedical Acss.).

Die bisherigen Methoden, Partikeln mittels kolloidaler Arzneistoffträger, wie Liposomen oder Nanokapseln, intravenös zu verabreichen, haben den Nachteil, daß sie nur die Verabreichung geringer Substanzmengen ermöglichen und teilweise aus nur langsam biotransformierbaren Materialien, wie Cyanoacrylaten bestehen.

Der Erfindung liegt die Aufgabe zu Grunde, für in physiologisch verträglichen, hydrophilen und lipophilen Medien sehr schwer lösliche Substanzen eine Rezeptur zu entwickeln, die gut verträglich ist, eine intravenöse Verabreichung hoher Dosen ermöglicht, für möglichst viele Substanzen anwendbar und zumindest kurzfristig stabil ist.

Die Aufgabe wird erfindungsgemäß durch eine pharmazeutische Formulierung gelöst, die aus einem dispersen System fest/flüssig/flüssig mit einer maximalen Partikelgröße von 5 µm besteht, das dadurch erhalten wurde, daß man bis zu 1,5 Gew. % (- z.B. durch Naßmahlung in einer Rührwerksmühle -) feinst vermahlener Substanz mit einer Korngröße von maximal 3 µm in 8-30 Gew. % Ölphase, die 1,0-4,5 Gew. % Emulgator, bezogen auf den Gesamtansatz enthält, dispergiert und diesen Ansatz mit 64-90 Gew. % aqua ad injektabilia emulgiert, isotonisiert und mittels eines Hochdruckhomogenisators homogenisiert.

Für die erfindungsgemäße Verarbeitung kommen in öligen und wäßrigen Medien schlecht lösliche Substanzen, wie beispielsweise bestimmte Cytostatika, Antibiotika und Spironolacton in Betracht. Die einzusetzenden Mengen hängen von verschiedenen Faktoren, u.a. Wirksamkeit der Substanz und Indikationsgebiet ab. Sie lassen sich daher nur schwer allgemein festlegen. In den meisten Fällen wird man jedoch vorzugsweise mehr als 0,03 Gew. % und nicht mehr als 1,5 Gew. % Substanz verwenden.

Als Ölphase können alle pharmazeutisch für Injektionspräparate verwendbaren lipophilen Medien, wie beispielsweise Baumwollsaatöl, Erdnußöl, Ethyloleat, Isopropylmyristat, Maisöl, mittelkettige Triglyceride, Olivenöl, Rizinusöl, Sojaöl und hydrierte flüssige pflanzliche Öle eingesetzt werden. Bevorzugt wird Miglyol 812 ® (gesättigte Triglyceride mit $C_8$-$C_{12}$ Fettsäuren) in Mengen von vorzugsweise (vzw.) 9 bis 20 Gew. % der Gesamtformulierung verwandt.

Als Emulgator oder Bestandteil einer Emulgatormischung können parenteral verträgliche Emulgatoren, wie Eilecithine, Sojalecithine, hydrierte Eilecithine, hydrierte Sojalecithine, Cholesterol, acetylierte Monoglyceride und Polyethylenglykol-Polypropylenglykol-Blockpolymerisate zur Anwendung kommen. Bevorzugt werden Sojalecithine in Mengen von vzw. 1,3 bis 3,0 Gew. % der Gesamtformulierung eingesetzt.

Zur Isotonisierung werden neben vorzugsweise monovalenten Elektrolyten, zum Beispiel NaCl, auch nichtionische Zusätze, wie beispielsweise Glycerol, Mannit, Sorbit und Xylit, verwendet. Vorzugsweise wird NaCl eingesetzt, die bevorzugt einzusetzende Menge liegt zwischen 0,5 und 0,81 Gew. % der Gesamtformulierung.

Zur Homogenisierung kommen grundsätzlich alle Verfahren, mit denen eine Partikelfeinheit kleiner 5 µm erreicht werden kann, ohne daß die Feststoffpartikel die Ölphase verlassen, in Frage. Da parenterale Emulsionen im Industriemaßstab mittels Hochdruckhomogenisierung auf die erforderliche Partikelfeinheit gebracht werden und bei Ultraschalleinwirkung eine Zersetzung der Wirkstoffe oder der Hilfsstoffe zu befürchten ist, kommt vorzugsweise die Hochdruckhomogenisierung zur Anwendung.

Es hat sich gezeigt, daß die Kombination von isotonisierendem Zusatz und Emulgator Einfluß darauf hat, ob die Wirkstoffpartikel in der Ölphase verbleiben. So hat sich herausgestellt, daß bei Verwendung von vorzugsweise monovalenten Elektrolyten, beispielsweise NaCl, als isotonisierendem Zusatz und Lecithin oder Lecithin und Cholesterol als Emulgator die Feststoffpartikel die Ölphase nicht verlassen, und die Partikel trotz des Elektrolytzusatzes im für intravenös zu verabreichende Emulsionen geforderten Größenbereich kleiner 5 μm liegen, obwohl - wie aus der Literatur bekannt ist - mit Lecithin stabilisierte Emulsionen durch Elektrolyte destabilisiert werden können (C.D. Black, N.G. Popovich, Drug Intell. Clin. Pharm., 15 , 185 (1981)). Andererseits war bei Einsatz von hydriertem Lecithin, vorzugsweise Epikuron 200H(®), der Zusatz eines nichtionischen Agens zur Isotonisierung günstiger. Emulgator und isotonisierende Substanz werden daher bei der erfindungsgemäßen Formulierung vorzugsweise so eingesetzt, daß Elektrolyte mit Lecithin oder Lecithin und Cholesterol und nichtionische isotonisierende Agenzien mit hydriertem Lecithin kombiniert werden.

Die besonderen Vorzüge der erfindungsgemäßen Rezeptur sind die gute Verträglichkeit durch den ausschließlichen Einsatz physiologisch gut verträglicher Substanzen, fehlende thermische Belastung der Substanzen während der Herstellung und Verhinderung von Zersetzungsreaktionen in der Wasserphase (A.J. Repta, Topics in Pharmaceutical Sciences, D.D. Breimer und P. Speiser ed. 1981, S. 131, Elsevier/North-Holland Biomedical Press.). Als allgemeine Basisrezeptur kann verwendet werden:

| Wirkstoff | | bis 1,5 Gew.%, | vzw. | bis 0,5 Gew. % |
|-----------|----|----------------|------|----------------|
| Ölphase | 8 | – 30 Gew.%, | vzw. | 9– 20 Gew. % |
| Emulgator | 1 | – 4,5 Gew.%, | vzw. | 1,3– 3 Gew. % |
| Wasserphase | 64 | – 90 Gew.%, | vzw. | 76,5–89,7 Gew. % |

Zur erfindungsgemäßen Herstellung lassen sich sowohl Lecithine mit hohem Anteil an ungesättigten Fettsäuren vorzugsweise Epikuron 170(®) (ein speziell aufgereinigtes Sojalecithin) dann aber vorzugsweise mit Elektrolytzusatz, als auch solche mit nur 10-30 % Anteil an ungesättigten Fettsäuren verarbeiten. Erstere haben den Vorteil der problemloseren Emulgierung und Homogenisierung.

Wirkstoffe, die in die erfindungsgemäße Rezeptur eingearbeitet werden, zeigen nach einer parenteralen Applikation einen deutlich meßbaren Effekt. Der Grad der Freisetzung ist von der Zusammensetzung des Emulgators abhängig. Die intravenöse Applikation der Partikel wird gut vertragen und somit ist die Rezeptur auch für die parenterale Applikation zu Screeningzwecken im Tierversuch geeignet.

In folgenden Beispielen wird die erfindungsgemäße Herstellung der Rezeptur im einzelnen erläutert:

Beispiel 1:

| | Ansatzgröße (g) |
|---|---|
| Dihydroxyanthrachinon | 0,200 |
| Miglyol 812[R] | 3,800 |
| Epikuron 170[R] | 0,480 |
| Cholesterol | 0,048 |
| 0,9% NaCl | 35,472 |
| | 40,000 |

Eine 10 %ige Suspension von Dihydroxyanthrachinon in Miglyol 812® wurde in einer Dyno-Mill Typ KDL (kontinuierlicher Betrieb, Umfangsgeschwindigkeit 10 m/s, Zeit 60 min, 0,3 mm Glaskugeln) auf eine Teilchengröße von weniger als 3 μm vermahlen. Eine die erforderliche Menge Dihydroxyanthrachinon enthaltende Menge der Suspension wurde in einem Erlenmeyerkolben mit Miglyol 812® auf die Hälfte der gesamten Ölphase ergänzt, und der Ansatz mit einer 24 %igen Lösung des Lecithins in Miglyol 812® aufgefüllt. Anschließend wurde zunächst Cholesterol und danach aqua ad injectabilia unter ständigem Rühren zugeführt und nach Beendigung des Emulgierens erfolgte der Kochsalzzusatz. Die Hochdruckhomogenisierung wurde in einer French Pressure cell (Millner, Lawrence, French, Science, 3 , 633, 1950), die zusätzlich mit einem Feindosierventil versehen war, durchgeführt.

Die Homogenisierung erfolgt bei einem Druck von 550 bar und 35 µm Spaltbreite.

Beispiel 2:

|  | **Ansatzgröße (g)** |
|---|---|
| S830544 | 0,0124 |
| Miglyol 812[R] | 3,9876 |
| Epikuron 170[R] | 0,48 |
| Cholesterol | 0,048 |
| NaCl (0,9 %) | 35,472 |
|  | 40,000 |

Die chemische Bezeichnung für S830544 ist 1,4 bis (dl-2,3 oxidopropoxi) anthrachinon. Herstellung wie bei Beispiel 1. Homogenisierung mit 320 bar, 150 µm Spalt und 3 Durchgängen.

Die Substanz wurde im Tierversuch auf cytostatische Wirkung untersucht. Es wurden Versuchsgruppen zu je 6 BDF1 Mäusen (18-20g) gebildet und diesen $10^6$ L1210 Leukämiezellen einen Tag vor Versuchsbeginn i.p. verabreicht. Während des 5-tägigen Versuches bekam jede Gruppe eine bestimmte Dosis einmal täglich i.p. appliziert.

Ausgewertet wurde die median survival time (MST) der einzelnen Versuchsgruppen. Eine Division durch die median survival time der Kontrollgruppe ohne Substanzapplikation multipliziert mit 100 ergab die prozentuale Lebensverlängerung. Werte größer 125 % waren Anzeichen für eine cytostatische Wirkung. Zur Beurteilung der Todesursache diente das Auftreten oder Fehlen einer Aszitis. Die Aszitis trat bedingt durch die L1210 Leukämiezellen auf, und erlaubte somit eine Differenzierung zwischen der Toxizität der Testsubstanz und der des Tumors.

| **Dosierung** | **MST/Kontr. %** | **Aszitis** |
|---|---|---|
| 7,75 mg/Körperg. | 114 | 4 von 6 |
| 4,65 mg/Körperg. | 143 | 5 von 6 |
| 1,55 mg/Körperg. | 129 | 6 von 6 |

Die Ergebnisse zeigen, daß die erfindungsgemäße Formulierung die problemlose Applikation therapeutisch relevanter Substanzmengen ohne weiteres ermöglicht und daß die inkorporierte Substanz in therapeutisch relevanten Konzentrationen aus der erfindungsgemäßen Formulierung freigesetzt wird. Für die mittlere Dosierung konnte eine deutlich über dem Wirksamkeitskriterium liegende Lebensverlängerung ermittelt werden. Mit steigender Dosis gewinnt die Toxizität der Substanz gegenüber der des Tumors an Bedeutung.

Vergleicht man die erhaltenen Ergebnisse mit denen früherer Versuche, so stellt man fest, daß mit der erfindungsgemäßen Formulierung die Wirkung bei viel geringeren Dosen eintritt.

EP 0 256 285 B1

| Dosierung | MST/Kontr. % | Aszitis |
|---|---|---|
| 1 x 1000 mg/kg Körperg. | 81 | 1 von 6 |
| 1 x 800 mg/kg Körperg. | 81 | 3 von 6 |
| 1 x 600 mg/kg Körperg. | 95 | 6 von 6 |
| 1 x 400 mg/kg Körperg. | 95 | 6 von 6 |
| 1 x 200 mg/kg Körperg. | 100 | 6 von 6 |

Zur Durchführung dieser Versuche wurde die Substanz vor der i.p. Applikation in einem tensidhaltigen Medium dispergiert und mittels Ultraschall homogenisiert.

Beispiel 3:

| | Ansatzgröße (g) |
|---|---|
| S827942 | 0,128 |
| Miglyol 812[R] | 3,872 |
| Epikuron 170[R] | 0,48 |
| Cholesterol | 0,048 |
| NaCL 0,9 % | 35,472 |
| | 40,000 |

Die chemische Bezeichnung für S827942 ist 3-Methyl-9-((2-Methoxy-4-Methylsulfonylamino)-Anilino)-Thieno <3,2-B> Chinolin-Lactat. Herstellung, Homogenisierungsbedingungen und Substanztestung analog Beispiel 2.

| Dosierung | MST/Kontr. % | Aszitis |
|---|---|---|
| 80 mg/kg Körperg. | 157 | 0 von 6 |
| 48 mg/kg Körperg. | 157 | 0 von 6 |
| 16 mg/kg Körperg. | 157 | 6 von 6 |
| 8 mg/kg Körperg. | 129 | 6 von 6 |
| 1,6 mg/kg Körperg. | 129 | 6 von 6 |

Beispiel 3 belegt ebenfalls, daß die eingearbeitete Substanz in wirksamen Konzentrationen freigesetzt wird. Ein Vergleich dieser Ergebnisse mit denen früherer Versuche zeigt, daß die cytostatische Wirkung bei erfindungsgemäßer Herstellung bei weitaus geringeren Dosen feststellbar ist.

5

| <u>Dosierung</u> | <u>MST/Kontr. %</u> | <u>Aszitis</u> |
|---|---|---|
| 1 x 800 mg/kg Körperg. | 138 | 4 von 10 |
| 1 x 700 mg/kg Körperg. | 149 | 7 von 10 |
| 1 x 600 mg/kg Körperg. | 154 | 9 von 10 |
| 1 x 500 mg/kg Körperg. | 147 | 10 von 10 |
| 1 x 400 mg/kg Körperg. | 147 | 10 von 10 |
| 1 x 300 mg/kg Körperg | 151 | 10 von 10 |

Die Präparation wurde analog zu Beispiel 2 durch Dispergierung der Substanz in einem tensidhaltigen Medium und anschließende Ultraschallhomogenisierung hergestellt.

Beispiel 4:

| | <u>Ansatzgröße (g)</u> |
|---|---|
| Spironolacton | 0,288 |
| Miglyol 812$^R$ | 7,712 |
| Epikuron 170$^R$ | 1,2 |
| Cholesterol | 0,12 |
| 0,9 % NaCl | <u>30,68</u> |
| | 40,000 |

Die Herstellung erfolgte analog zu Beispiel 1. Homogenisierung mit 660 bar, 150 $\mu$m Spalt und 3 Durchgängen.

Die Zubereitung wurde im Tierversuch auf ihre diuretische Wirkung untersucht. Es wurden Versuchsgruppen zu je 3 Lewisratten (160 - 180 g) gebildet und diese in Stoffwechselkäfigen untergebracht. Während des 4-tägigen Versuches erhielten die Tiere Nahrung und Wasser nach Bedarf und alle 24 Stunden wurde die ausgeschiedene Urinmenge gemessen. Zur besseren Beurteilung der Ergebnisse wurden verschiedene Kontrollgruppen gebildet:
- ohne Applikation
- Leergalenik i.p.
- Leergalenik i.v.

| Zubereitung | Dosierung mg/kg Körperg. | Urinausscheidung ml/Ratte/h | | | |
|---|---|---|---|---|---|
| | | 0–24 | 24–48 | 48–72 | 72–96 |
| Spironolacton | 25 i.p. | 0,42 | 0,44 | 0,28 | 0,3 |
| Spironolacton | 50 i.p. | 0,8 | 0,58 | 0,55 | 0,56 |
| Leergalenik | 1 ml i.p. | 0,16 | 0,2 | 0,25 | 0,28 |
| Spironolacton | 50 i.v. | 0,6 | 0,55 | 0,39 | 0,3 |
| Leergalenik | 1 ml i.v. | 0,47 | 0,36 | 0,38 | 0,33 |
| ohne Applikation | | 0,33 | 0,25 | 0,16 | 0,25 |

| Zubereitung | Dosierung (mg/kg Körperg.) | kumulative Urinausscheidung pro Ratte nach 96 h (ml) |
|---|---|---|
| Spironolacton | 25 i.p. | 34,56 |
| Spironolacton | 50 i.p. | 60,48 |
| Leergalenik | 1 ml i.p. | 21,12 |
| Spironolacton | 50 i.v. | 44,16 |
| Leergalenik | 1 ml i.v. | 35,1 (90 h) |
| ohne Applikation | | 24 |

Spironolacton wird in therapeutisch relevanter Konzentration aus der erfindungsgemäßen Formulierung freigesetzt. Die ermittelten Daten belegen, daß nach i.p. Applikation eine stärkere und längere diuretische Wirkung zu verzeichnen ist, als nach i.v. Applikation. Die ausgeschiedene Urinmenge wird gegenüber der Gruppe mit Leergalenik bei i.v. Applikation um ca. 25 % erhöht, während bei i.p. Applikation eine Steigerung von ca. 185 % feststellbar ist.

Beispiel 5:

|  | Ansatzgröße (g) |
|---|---|
| Spironolacton | 0,288 |
| Miglyol 812$^R$ | 7,712 |
| Epikuron 200H$^R$ | 0,96 |
| Epikuron 170$^R$ | 0,24 |
| 2,5% Glycerol | 30,8 |
|  | 40,000 |

Die Herstellung erfolgte analog zu Beispiel 1. Homogeniierung mit 300 bar, 100 $\mu$m Spaltbreite und 3 Durchgängen.

Der Tierversuch wurde unter den gleichen Bedingungen wie bei Beispiel 4 durchgeführt.

| Zubereitung | Dosierung (mg/kg Körperg.) | Urinausscheidung ml/Ratte/h | | | |
|---|---|---|---|---|---|
|  |  | 0-24 | 24-48 | 48-72 | 72-90 |
| Spironolacton | 50 i.p. | 0,5 | 0,42 | 0,36 | 0,33 |
| Leergalenik | 1 ml i.p. | 0,28 | 0,31 | 0,32 | 0,3 |
| Spironolacton | 50 i.v. | 0,44 | 0,42 | 0,48 | 0,63 |
| Leergalenik i.v. | 1 ml i.v. | 0,47 | 0,36 | 0,36 | 0,37 |
| ohne Applikation |  | siehe Beispiel 4 | | | |

| Zubereitung | Dosierung (mg/kg Körperg.) | kumulative Urinausscheidung pro Ratte nach 90 h (ml) |
|---|---|---|
| Spironolacton | 50 i.p. | 36 |
| Leergalenik | 1 ml i.p. | 27 |
| Spironolacton | 50 i.v. | 44,1 |
| Leergalenik i.v. | 1 ml i.v. | 35,1 |
| ohne Applikation |  | siehe Beispiel 4 |

Auch in Beispiel 5 ist eine Diuresesteigerung gegenüber den Kontrollgruppen feststellbar. Es ist jedoch auffällig, daß die Diuresesteigerung bei i.p. Applikation gegenüber Beispiel 4 deutlich geringer ist, und daß nach i.v. Applikation das Wirkungsmaximum verzögert auftritt. Diese Effekte sind auf die Variation der

EP 0 256 285 B1

Lecithinkomponente zurückzuführen. Offensichtlich erfolgt die Freisetzung um so schneller und vollständiger, je mehr ungesättigte Fettsäuren die Lecithinkomponente enthält.

## Ansprüche

1. Pharmazeutische Formulierung zur parenteralen Verabreichung sowohl in hydrophilen, als auch in lipophilen Medien schwer löslicher Substanzen, bestehend aus einem dispersen System fest/flüssig/flüssig mit einer maximalen Partikelgröße von 5 $\mu$m, das dadurch erhalten wurde, daß man bis zu 1,5 Gew. % feinst vermahlener Substanz mit einer Korngröße von maximal 3 $\mu$m in 8-30 Gew. % Ölphase, die 1,0-4,5 Gew. % Emulgator enthält, dispergiert und diesen Ansatz mit 64-90 Gew. % agua ad injektabilia emulgiert, isotonisiert und mittels eines Hochdruckhomogenisators homogenisiert, wobei alle Prozentangaben auf den Gesamtansatz bezogen sind.

2. Pharmazeutische Formulierung nach Anspruch 1, dadurch gekennzeichnet, daß als isotonisierende Substanz ein monovalenter Elektrolyt und als Emulgator Lecithin oder Lecithin und Cholesterol eingesetzt werden.

3. Pharmazeutische Zubereitung nach Anspruch 1, dadurch gekennzeichnet, daß als isotonisierende Substanz eine nichtionische Substanz und als Emulgator hydriertes Lecithin eingesetzt werden.

4. Verfahren zur Herstellung einer pharmazeutischen Formulierung zur parenteralen Verabreichung sowohl in hydrophilen, als auch in lipophilen Medien schwer löslicher Substanzen, dadurch gekennzeichnet, daß man bis zu 1,5 Gew. % feinst vermahlene Substanz mit einem Korngrößendurchmesser von maximal 3 $\mu$m in 8-30 Gew. % Ölphase, die 1,0-4,5 Gew. % Emulgator enthält, dispergiert und diesen Ansatz mit 64-90 Gew. % agua ad injektabilia emulgiert, wobei alle Prozentangaben auf den Gesamtansatz bezogen sind, isotonisiert und mittels eines Hochdruckhomogenisators homogenisiert, so daß als Produkt ein disperses System fest/flüssig/flüssig mit einem maximalen Partikeldurchmesser von 5 $\mu$m entsteht.

Patentanspruch für folgende Vertragsstaaten : GR, ES

1. Verfahren zur Herstellung einer pharmazeutischen Formulierung zur parenteralen Verabreichung schwer löslicher Substanzen, dadurch gekennzeichnet, daß bis zu 1,5 Gew. % feinst vermahlene Substanz mit einem Korngrößendurchmesser von maximal 3 $\mu$m in 8-30 Gew. % Ölphase, die 1,0-4,5 Gew. % Emulgator, bezogen auf den Gesamtansatz, enthält, dispergiert und diesen Ansatz mit 64-90 Gew. % agua ad injektabilia emulgiert, isotonisiert und mittels eines Hochdruckhomogenisators homogenisiert, so daß als Produkt ein disperses System fest/flüssig/flüssig mit einem maximalen Partikeldurchmesser von 5 $\mu$m entsteht.

## Claims

1. A pharmaceutical formulation for the parenteral administration of substances which are sparingly soluble both in hydrophilic and in lipophilic media, composed of a solid/liquid/liquid disperse system which has a maximum particle size of 5 $\mu$m and which has been obtained by dispersing up to 1.5% by weight of very finely ground substance with a particle size not exceeding 3 $\mu$m in 8-30% by weight of oil phase which contains 1.0-4.5% by weight of emulsifier, and emulsifying this mixture with 64-90% by weight of water for injection, rendering isotonic, and homogenizing using a high-pressure homogenizer, all percentages being based on the total mixture.

2. A pharmaceutical formulation as claimed in claim 1, wherein the substance used for rendering isotonic is a monovalent electrolyte, and the emulsifier used is lecithin or lecithin and cholesterol.

3. A pharmaceutical preparation as claimed in claim 1, wherein the substance used for rendering isotonic is a non-ionic substance, and the emulsifier used is hydrogenated lecithin.

9

4. A process for the preparation of a pharmaceutical formulation for the parenteral administration of substances which are sparingly soluble both in hydrophilic and in lipophilic media, which comprises dispersing up to 1.5% by weight of very finely ground substance with a particle diameter not exceeding 3 μm in 8-30% by weight of oil phase which contains 1.0-4.5% by weight of emulsifier, and emulsifying this mixture with 64-90% by weight of water for injection, all percentages being based on the total mixture, rendering isotonic, and homogenizing using a high-pressure homogenizer, so that the resultant product is a solid/liquid/liquid disperse system with a maximum particle diameter of 5 μm.

Claim for the following Contracting States : GR, ES

1. A process for the preparation of a pharmaceutical formulation for the parenteral administration of substances which are sparingly soluble both in hydrophilic and in lipophilic media, which comprises dispersing up to 1.5% by weight of very finely ground substance with a particle diameter not exceeding 3 μm in 8-30% by weight of oil phase which contains 1.0-4.5% by weight of emulsifier, and emulsifying this mixture with 64-90% by weight of water for injection, all percentages being based on the total mixture, rendering isotonic, and homogenizing using a high-pressure homogenizer, so that the resultant product is a solid/liquid/liquid disperse system with a maximum particle diameter of 5 μm.

## Revendications

1. Formulation pharmaceutique pour l'administration parentérale de substances difficilement solubles aussi bien dans des milieux hydrophiles que lipophiles, constituée d'un système dispersé solide-liquide-liquide à particules de granulométrie maximale 5 μm, que l'on obtient en dispersant, dans 8 à 30% en poids d'une phase huileuse renfermant 1,0-4,5% en poids d'émulsifiant, jusqu'a 1,5% en poids de substance très finement broyée, de granulométrie maximale 3 μm, et en émulsifiant le tout avec 64-90% en poids d'eau pour injection, puis en rendant l'émulsion isotonique et en l'homogénéisant à l'aide d'un homogénéisateur sous haute pression, tous les pourcentages étant rapportés au poids total.

2. Formulation pharmaceutique selon la revendication 1, caratérisée en ce l'on utilise comme substance isotonisante un électrolyte monovalent et comme émulsifiant de la léchithine ou de la lécithine et du cholestérol.

3. Formulation pharmaceutique selon la revendication 1, caractérisée en ce l'on utilise comme substance isotonisante une substance non-ionique et comme émulsifiant de la lécithine hydogénée.

4. Procédé de préparation d'une formulation pharmaceutique pour l'administration parentérale de substances difficilement solubles aussi bien dans des milieu hydrophiles que lipophiles, caractérisé en ce que l'on disperse, dans 8 à 30% en poids d'une phase huileuse renfermant 1,0-4,5% en poids d'émulsifiant, jusqu'à 1,5% en poids de substance très finement broyée, de granulométrie maximale 3μm, en ce que l'on émulsifie le tout avec 64-90% en poids d'eau pour injection, tous les pourcentages étant rapportés au poids total, en ce que l'on rend l'émulsion isotonique et en ce qu'on l'homogénéise à l'aide d'un homogénéisateur sous haute pression, de manière à ce qu'il se forme, comme produit, un système dispersé solide-liquide-liquide à particules de diamètre maximal 5 μm.

Revendications pour les Etats contractants suivants : GR, ES

1. Procédé de préparation d'une formulation pharmaceutique pour l'administration parentérale de substances difficilement solubles aussi bien dans des milieux hydrophiles que lipophiles, caractérisé en ce que l'on disperse, dans 8 à 30% en poids d'une phase huileuse renfermant 1,0-4,5% en poids d'émulsifiant, jusqu'à 1,5% en poids de substance très finement broyée, de granulométrie maximale 3 μm, en ce que l'on émulsifie le tout avec 64-90% aen poids d'eau pour injection, tous les pourcentages étant rapportés au poids total, en ce que l'on rend l'émulsion isotonique et en ce qu'on l'homogénéise à l'aide d'un homogénéisateur sous haute pression, de manière à ce qu'il se forme, comme produit, un système dispersé solide-liquide-liquide à particules de diamètre maximal 5 μm.